(19) Europäisches Patentamt
European Patent Office
Office européen des brevets

(11) Veröffentlichungsnummer: **0 144 980**
**B1**

(12) **EUROPÄISCHE PATENTSCHRIFT**

(45) Veröffentlichungstag der Patentschrift: **12.07.89**

(51) Int. Cl.⁴: **C 07 C 99/00,** C 07 C 99/12 // C07C101/04, C07C101/18

(21) Anmeldenummer: **84114853.9**

(22) Anmeldetag: **06.12.84**

(54) **Verfahren zur Herstellung von optisch aktiven 3-Aminocarbonsäure (estern).**

(30) Priorität: **07.12.83 CH 6532/83**

(43) Veröffentlichungstag der Anmeldung: **19.06.85 Patentblatt 85/25**

(45) Bekanntmachung des Hinweises auf die Patenterteilung: **12.07.89 Patentblatt 89/28**

(84) Benannte Vertragsstaaten: **BE CH DE FR GB IT LI NL**

(56) Entgegenhaltungen:
**EP-A-0 062 840**
**DE-B-1 073 501**

**CHEMICAL ABSTRACTS, Band 89, Nr. 23, 4. Dezember 1978, Seite 668, Zusammenfassung Nr. 197899s, Columbus, Ohio, US; M. FURUKAWA et al.: "Asymmetric syntheses of beta-amino acids and aspartic acid by the Reformatskii reaction", & CHEM. PHARM. BULL. 1978, 26(1), 260-3**
**CHEMICAL ABSTRACTS, Band 92, Nr. 15, 14. April 1980, Seite 741, Columbus, Ohio, US; M. FURUKAWA et al.: "Asymmetric syntheses of beta-amino acids by the reduction of enamines", & CHEM. PHARM. BULL. 1979, 27(9), 2223-6**

(73) Patentinhaber: **LONZA AG, Gampel/Wallis (CH)**

(72) Erfinder: **Jolidon, Synèse, Dr., Weidenstrasse 42, CH-4106 Therwil (CH)**
Erfinder: **Meul, Thomas, Dr., Balfrinstrasse 21, CH-3930 Visp (CH)**

(74) Vertreter: **Weinhold, Peter, Dr., Patentanwälte Dr. V. Schmied- Kowarzik Dipl.- Ing. G. Dannenberg Dr. P. Weinhold Dr. D. Gudel Dipl.- Ing. S. Schubert Dr. P. Barz Siegfriedstrasse 8, D-8000 München 40 (DE)**

**EP 0 144 980 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die vorliegende Erfindung betrifft ein Verfahren zur Herstellung von optisch aktiven 3-Aminocarbonsäureestern aus den entsprechenden β-Ketosäureestern und der entsprechenden Säuren durch Verseifung der Ester.

Diese Verbindungen sind Grundbausteine, welche in ihrer β-Lactamform in zahlreichen Antibiotika und in optisch aktiver Form in natürlich vorkommenden Peptiden enthalten sind.

Es ist bekannt, optisch aktive β-Aminosäuren durch Hydrogenolyse von chiralen Enaminen mit 10 % Palladiumhydroxid auf Kohle oder mit Natriumcyanoborhydrid in optischen Reinheiten von 3-28 % und Ausbeuten von 11 - 32 % herzustellen [Furukawa et al, Chem. Pharm.Bull. 27(9) 2223 - 2226 (1979)]. Als nachteilig erwiesen sich bei diesem Verfahren die schlechten optischen Reinheiten.

Der Erfindung liegt die Aufgabe zugrunde, ein Verfahren zu entwickeln, das die Herstellung optisch aktiver 3-Aminocarbonsäureester und entsprechend die Säuren durch Verseifung in hohen optischen Ausbeuten erlaubt.

Erfindungsgemäss wird dies nach dem Patentanspruch 1 erreicht, indem man einen β-Ketosäureester mit einem chiralen Amin in das entsprechende Enamin überführt. Aufgrund des eingebauten chiralen Hilfsstoffes nimmt die nachfolgende Hydrierung in Gegenwart eines Platinkatalysators zu N-substituierten Aminosäureestern einen diastereoselektiven Verlauf. Die Trennung des anfallenden diastereomeren Gemisches vollzieht sich über die entsprechenden, mit Salzsäuregas in wasserfreien Lösungsmitteln herstellbaren Hydrochloride, wobei man deren unterschiedliches Löslichkeitsverhalten ausnutzt.

Darauffolgende Neutralisation des gewünschten Hydrochlorids und Abspaltung des N-Substituenten durch Hydrogenolyse in Gegenwart eines Palladiumkatalysators führt zum gewünschten optisch aktiven 3-Aminocarbonsäureester, der sich durch Verseifung in die entsprechende, optisch aktive 3-Aminocarbonsäure umwandeln lässt.

Bei dieser Reaktionsfolge ist es von entscheidender Bedeutung, dass man zur Hydrierung der Enamin-Doppelbindung den selektiveren Platinkatalysator verwendet, der eine gleichzeitige Abspaltung der chiralen Hilfsgruppe vermeidet. Mit Hilfe des Palladiumkatalysators gelingt die Entfernung der chiralen Hilfsgruppe, nachdem auf der Stufe der Hydrochloride das gewünschte Diastereomer angereichert wurde.

Als β-Ketosäureester kommen Verbindungen der Formel

$$R - CH_2 - \overset{\overset{\displaystyle O}{\|}}{C} - CH_2 - \overset{\overset{\displaystyle O}{\|}}{C}O - R_1$$

in Frage, worin R = H oder Alkyl, geradkettig oder verzweigt, substituiert oder unsubstituiert, oder Benzyl, substituiert oder unsubstituiert, und $R_1$ = Alkyl, geradkettig oder verzweigt, substituiert oder unsubstituiert, oder Benzyl, substituiert oder unsubstituiert, bedeutet.

Vorzugsweise Verbindungen sind solche mit R = H, oder Alkyl mit 1 bis 4 C-Atomen oder Benzyl und $R_1$ = Alkyl mit 1 bis 4 C-Atomen.

Die Umsetzung zum Enamin wird mit Vorteil mit optisch aktivem 1-Phenylethylamin als chiralem Amin durchgeführt. Dabei verfährt man in üblicher Weise in einem Lösungsmittel in Gegenwart saurer Katalysatoren.

Die sich anschliessende Hydrierung des Enamins zum N-substituierten Aminosäureester wird in Gegenwart eines Platinkatalysators durchgeführt, der 2 bis 10 %, vorzugsweise 5 % Platin auf einem Trägermaterial wie Bimsstein, Kohle, Silicagel oder Tonerde, mit Vorteil aber Kohle, aufweisen kann.

Die Katalysatormenge kann gering gehalten werden. Sie beträgt zweckmässigerweise 3 bis 5 Gew.-%, vorzugsweise 4 Gew.-%, bezogen auf eingesetztes Enamin.

Die Umsetzungstemperatur wählt man zweckmässig zwischen 20 bis 40°C, vorzugsweise Raumtemperatur.

Die Hydrierung wird bevorzugt bei Normaldruck durchgeführt.

Nach dieser Reduktion entsteht ein Diastereomergemisch aus RRund RS- oder SS- und SR-Diastereomer.

Die darauffolgende Umsetzung zu den entsprechenden diastereomeren Hydrochloriden wird zweckmässig mit Überschuss an Salzsäuregas in einem wasserfreien Lösungsmittel, wie Ether, Tetrahydrofuran oder Dioxan, vorzugsweise in Ether durchgeführt.

Die diastereomeren Hydrochloride werden dann zur Trennung des Diastereomeren-Gemischs verwendet, was zweckmäßig durch Umkristallisation in geeigneten Lösungsmitteln bzw. Lösungsmittelgemischen erfolgt. Dies können aliphatische Alkohole mit $C_1$ bis $C_5$-Kette, wie z. B. Methanol, Ethanol oder Butanol, sein, wobei im Falle des Methanols durch Zusatz von Ketonen oder Carbonsäureestern, wie Aceton oder Essigester, der Kristallisationsvorgang eingeleitet werden kann. Auch Gemische von chlorierten Kohlenwasserstoffen, wie Chloroform oder Methylenchlorid, mit Ketonen oder Carbonsäureestern sind geeignet. Diese Prozedur kann zwecks Erzielung eines reinen Diastereomers mehrmals wiederholt werden. In der Regel wird nach 2-maliger Umkristallisation eine Anreicherung grösser als 95 % eines Diastereomers erreicht.

Das weitgehend optisch reine Hydrochlorid kann auf übliche Weise durch Zugabe einer geeigneten Base, wie z. B. wässrige Ammoniaklösung, neutralisiert werden.

Anschliessend wird der vorliegende N-substituierte 3-Aminocarbonsäureester einer Hydrogenolyse unterzogen.

Man arbeitet dazu in Gegenwart eines Palladiumkatalysators, der 2 bis 10 %, vorzugsweise 5 % Palladium, auf Trägermaterialien wie Bimsstein, Kohle oder Tonerde, vorzugsweise Kohle, besitzen kann, wobei

vorzugsweise alkoholische Lösungsmittel wie Methanol, Ethanol oder Propanol verwendet werden können.

Die Katalysatormenge beträgt zweckmässig 9 bis 15 Gew.-%, vorzugsweise 9 bis 12 Gew.-%, bezogen auf eingesetzten N-substituierten 3-Aminocarbonsäureester.

Die Umsetzungstemperatur wählt man zweckmässig zwischen 20 bis 40° C, vorzugsweise Raumtemperatur.

Der Hydrierdruck kann zwischen 1 x $10^5$ bis 1 x $10^6$ Pa liegen, vorzugsweise arbeitet man unter Normaldruck.

Nach üblicher Aufarbeitung durch Trennen vom Katalysator, Eindampfen der Lösung und Destillation, kann man den optisch aktiven 3-Aminocarbonsäureester isolieren. Zur Herstellung der freien, optisch aktiven Aminocarbonsäuren wird der 3-Aminocarbonsäureester in einem letzten Schritt verseift. Dies geschieht durch Erhitzen des Aminoesters auf Temperaturen von zweckmässig 80 bis 90° C, vorzugsweise 85° C, in saurem Medium. Als saures Medium werden zweckmässig Mineralsäuren, wie konzentrierte Salzsäure, angewendet. Die Umsetzungszeit beträgt zweckmäßiq zwischen 3 und 6 Stunden. Nach üblicher Aufarbeitung, z. B. in der Folge Abdampfen des sauren Mediums, Aufnahme des Rückstandes in einem alkoholischen Lösungsmittel, Neutralisation des Hydrochlorids durch Zugabe eines organischen Amins, können die freien 3-Aminocarbonsäuren erhalten werden. Die auf diese Weise hergestellten optisch aktiven 3-Aminocarbon-säure liegen in einer optischen Reinheit von grösser als 90 % vor.

Das Verfahren der Erfindung wird in Beispiel 1 anhand der Herstellung von R-(-)-3-Aminobuttersäure demonstriert. Acetessigsäuremethylester wird mit R-(+)-1-Phenylethylamin und Essigsäure in Toluol umgesetzt.

Man erhält nach üblicher Aufarbeitung durch Abdampfen des Lösungsmittels und Hochvakuumdestillation das entsprechende Enamin.

Dieses wird darauffolgend wie beschrieben hydriert, zweckmässig in Methanol als Lösungsmittel.

Nach Aufarbeiten durch Abdampfen des Lösungsmittels und Hochvakuumdestillation wird der entsprechende 3-(1'-Methylbenzylamino)-butansäuremethylester als Diastereomergemisch erhalten, wobei eine Anreicherung des RR-Isomers festzustellen ist.

Für die darauffolgende Hydrochloridbildung löst man das Diastereomergemisch vorzugsweise in Ether und sättigt es anschliessend mit Salzsäuregas.

Nach der Filtration und Trocknung des ausgefallenen Kristallbreies kann das reine Hydrochlorid wiederum als Diastereomergemisch erhalten werden.

Zur Trennung dieses Diastereomergemisches werden die Hydrochloride heiss aus Methanol/Aceton umkristallisiert.

Nach zweimaliger Umkristallisation erzielt man eine Anreicherung des RR-Isomers von mindestens 95 %.

Das weitgehend reine Diastereomer wird zur Erzielung des enantiomeren Endprodukts in einem ersten Schritt durch Zugabe von wässriger Ammoniaklösung neutralisiert. Das freie Amin kann dann nach zweckmässiger Isolation durch Extraktion oder Destillation zur Abspaltung des Methylbenzylsubstituenten einer Hydrogenolyse in Gegenwart eines Palladiumkatalysators unterzogen werden. Die sich anschliessende Destillation ergibt den R-(-)-3-Aminobuttersäuremethylester, der zur R-(-)-3-Aminobuttersäure verseift werden kann. Die optische Reinheit der so hergestellten R-(-)-3-Aminobuttersäure ist grösser als 90 %.

Die gleiche Synthese kann auch mit S-(-)-1-Phenylethylamin durchgeführt werden. Man erhält dann in der gleichen optischen Reinheit den S-(+)-3-Aminobuttersäuremethylester, resp. die S-(+)-3-Aminobuttersäure.

Die Beispiele 2 bis 4 zeigen das erfindungsgemässe Verfahren für die Herstellung der Aminopentan-, Aminohexan- sowie Aminobenzylbuttersäureestern.

**Beispiel 1**

**Herstellung von optisch aktiven 3-Aminobuttersäuren**

1.1 Herstellung von 3-(1'-Methylbenzylamino)-2Z-butensäureestern

a) Eine Mischung aus 54,6 g (0,47 Mol) Acetessigsäuremethylester, 54,5 g (0,45 Mol) R-(+)-1-Phenylethylamin und 1,0 g Essigsäure wurde in 150 ml Toluol 2 Std am Rückfluss gehalten. Das Reaktionswasser (8,3 ml; 102 % der Theorie) wurde über einen Wasserabscheider entfernt. Anschliessend wurde die Lösung im Rotationsverdampfer eingedampft und im Hochvakuum destilliert. Dabei isolierte man als Hauptfraktion ($Sdp._{0,4}$:108° C) 84,6 g (86 % R-(-)-(1'-Methylbenzylamino)-2Z-butensäuremethylester als farbloses Oel. $[\alpha]_D^{25}$ (c = 2; EtOH) = -545°.

UV (in EtOH): $\lambda_{max.}$ = 287 nm
IR (Film): 3280 (NH), 1655 ($COOCH_3$), 1605/1492 (Ar), 1270 ($O-CH_3$), 760/700 (5 benachbarte arom. H's)
NMR ($CDCl_3$): 1,50 (d; J = 7 Hz; $CH_3$-CH; 3H); 1,76 (s; $CH_3$-C= ; 3H); 3,64 (s; $OCH_3$; 3H); 4,47 (s; =CH-; IH); 4,62 (qaxd; J = 7 Hz; Ph-CH; IH); 7,26 (s; Aromat; 5H); 8,90 (s breit; NH; IH).

b) Analog wurde aus Acetessigsäuremethylester und S-(-)-1-Phenylethylamin der entsprechende S-(+)-3-(1'-Methylbenzylamino)-2Z-butensäuremethylester hergestellt.

Ausbeute: 51 %; $Sdp._{0,8}$:130° C (teilweise Polymerisation während der Destillation); $[\alpha]_D^{25}$ (c = 2; EtOH) = +512°.

c) Aus Acetessigsäureethylester und R-(+)-1-Phenylethylamin wurde der R-(-)-3-(1'-Methylbenzylamino)-2Z-butensäureethylester hergestellt.

Ausbeute: 40 %; $Sdp._{0,6}$: 120°C (teilweise Polymerisation während der Destillation); $[\alpha]_D^{25}$ (c = 2; EtOH) = -447°

UV (in EtOH): $\lambda_{max.}$ = 288 nm
IR (Film): 3280 (NH); 1650 ($COOCH_3$); 1610/1492 (Ar); 1265 ($OCH_3$); 760/700 (5 benachbarte arom. H's);
NMR ($CDCl_3$): 1,26 (t; J = 7,5 Hz; $CH_3$-$CH_2$-; 3H); 1,49 (d; J = 7 Hz; $CH_3$-CH-; 3H); 1,74 (s; $CH_3$-C=; 3H); 4,08 (qa; J = 7,5 Hz; $CH_2$-$CH_3$; 2H); 4,44 (s; -CH=; 1H); 4,58 (qaxd; Ph-CH; 1H); 7,20 (s; Aromat; 5H), 8,85 (s breit; NH; 1H)

d) Aus Acetessigsäure-t-butylester und R-(+)-1-Phenylethylamin wurde der R-(-)-3-(1'Methylbenzylamino)-2Z-butensäure-t- butylester hergestellt.

Ausbeute: 79 %; $Sdp._{0,4}$: 115°C; $[\alpha]^{25}_D$ (c = 2; EtOH) = -528°

IR (Film): 3280 (NH) 1650 ($COOCH_3$); 1610/1490 (Aromat) 750/700 (5 benachbarte arom. H's)
NMR ($CDCl_3$): 1,47 (s; t-Bu; 9H); 1,49 (d; J = 7 Hz; $CH_3$-CH; 3H); 1,71 (s; $CH_3$-C=; 3H); 4,37 (5; =CH-; 1H); 4,55 (qaxd; Ph-CH-; 1H); 7,19 (s; Aromat; 5H); 8,60 (s breit; NH; 1H)

1.2 Herstellung von 3(1'-Methylbenzylamino)-butansäureestern

a) 25,0 g R(-)-Methylester (0,114 Mol) wurden in 100 ml Methanol gelöst, mit 1,0 g Platin 5 % auf Kohle versetzt und bei Raumtemperatur und Normaldruck hydriert. Die Wasserstoffaufnahme war nach 10 Std beendet und betrug 96 % der Theorie. Der Katalyt wurde abfiltriert, die Lösung im Rotationsverdampfer eingedampft und der Rückstand im Hochvakuum destilliert. Dabei isolierte man als Hauptfraktion mit einem $Sdp._{0,5}$ : 88 - 92°C 23,2 g (92 %) 3-(1'-Methylbenzylamino)-butansäuremethylester als Diastereomergemisch (laut NMR: 73 % 1'R, 3R und 27 % 1'R, 3S-Isomer).

IR (Film): 3430/3360 (NH); 1738 ($COOCH_3$); 1600 (Aromat); 761/702 (5 benachbarte arom. H's)
NMR ($CDCl_3$): 1,02 (d; J = 6 Hz; $CH_3$-CH-$CH_2$; 3H); 1,31 (d; J = 7 Hz; Ph-CH-$CH_3$; 3H); 1,53 (s breit; NH; 1H); 2,30 (d; J = 5 Hz; -$CH_2$- von RS-Isomer; 0,54 H); 2,42 (d; J = 5 Hz; -$CH_2$ von RR-Isomer; 1,46 H); 2,95 (qaxdxd; $CH_3$-CH-$CH_2$; 1H); 3,60 (s; $OCH_3$ von RS-Isomer; 0,81 H); 3,63 (s; $OCH_3$ von RR-Isomer; 2,19 H); 3,87 (qaxd; Ph-CH-; 1H); 7,28 (s; Aromat; 5H)

b) Analog wurde aus dem S-(+)-Methylester der entsprechende 3-(1'-Methylbenzylamino)-butansäuremethylester als Diastereomergemisch erhalten (laut NMR; 73 % 1'S, 3S und 27 % 1'S, 3R-Isomer).

Ausbeute 79 %.

c) Ausgehend vom R-(-)-Ethylester erhielt man 3-(1'-Methylbenzylamino)-butansäureethylester als Diastereomergemisch (laut NMR; ca. 65 % 1'R, 3R und ca. 35 % 1'R, 3S-Isomer).

Ausbeute: 76 %; $Sdp._{0,5}$ : 100 - 110°C.

IR (Film); 3430/3330 (NH); 1732 ($COOCH_3$); 1600 (Aromat); 760/700 (5 benachbarte arom. H's)
NMR ($CDCl_3$): 1,03 (d; J = 7 Hz; $CH_3$-CH-$CH_2$; 3H); 1,11 (t; J = 7 Hz, $CH_3$-$CH_2$; 3H); 1,31 (d; J = 6,5 Hz; Ph-CH-$CH_3$; 3H); 1,60 (s breit; 1H); 2,28 (d; J = 5 Hz; -$CH_2$-COO- von RS-Isomer; 0 7 H); 2,40 (d; J = 5 HZ; -$CH_2$-COO- von RR-Isomer; 1,3 H); 2,95 (qaxdxd; $CH_3$-CH-$CH_2$; 1H); 3,92 (qaxdxd; Ph-CH; 1H); 4,11 (qa; -$CH_2$-$CH_3$; 2H); 7,25 (s; Aromat; 5H)

d) Aus dem R-(-)-t-Butylester erhielt man 3-(1'-Methylbenzylamino)-butansäure-t-butylester als Diastereomergemisch (laut NMR: 73 % 1'R, 3R und 27 % 1'R, 3S-Isomer).

Ausbeute: 93 %; $Sdp._{0,5}$ : 95 - 97°C.

IR (Film): 3430/3240 (NH); 1730 ($COOCH_3$); 1600 (Aromat); 760/700 (5 benachbarte arom H's)
NMR ($CDCl_3$): 1,02 (d; J = 7 Hz; $CH_3$-CH-$CH_2$; 3H); 1,31 (d; J = 6,5 Hz; Ph-CH-$CH_3$; 3H); 1,44 (s; t-Bu des RS-Isomers; 2,43 H); 1,45 (s; t-Bu des RR-Isomeren; 6,57 H); 2,11 (d; J = 5 Hz; -$CH_2$- des RS-Isomers; 0,54 H); 2,28 (d; J = 5 Hz; -$CH_2$ des RR-Isomers; 1,46 H); 2,90 (qaxdxd; $CH_3$-CH-$CH_2$; 1H); 3,86 (qa; $CH_3$-CH-Ph; 1H); 7,23 (s; Aromat; 5H)

1.3 Herstellung und Umkristallisation der Hydrochloride von 3-(1'-Methylbenzylamino)-butansäureestern

a) 20,0 g (90,4 mMol) des rohen Diastereomergemisches (73 % RR und 27 % RS) des Methylesters wurden in 300 ml Ether gegeben und die Lösung unter Eiskühlung mit gasförmiger Salzsäure gesättigt. Der Kristallbrei wurde abgenutscht und getrocknet. Man isolierte schliesslich 23,3 g (100 %) des Hydrochlorids von 3-(1'-Methylbenzylamino)-butansäuremethylesters als Diastereomergemisch (Smp = 195 - 198°C). 16,0 g (62,1 mMol) dieses Hydrochlorids wurden in 32 ml Methanol heiss gelöst. Durch langsame Zugabe von 80 ml Aceton wurde das angereicherte RR-Isomere zur Kristallisation gebracht. Abnutschen und Trocknen ergaben 8,55 g (53 %) farblose Kristalle des RR-Hydrochlorids (Smp. = 210 - 212°C).

Nochmalige Umkristallisation nach dem gleichen Verfahren lieferte schliesslich ein RR-Hydrochlorid mit

einem Smp. von 225 - 227° C.

1.4 Herstellung von 3-Aminobuttersäureestern

a) 8,35 g (32,4 mMol) des zweimal umkristallisierten Hydrochlorids des RR-Methylesters wurden in 50 ml Wasser gegeben, mit Ammoniak alkalisch gestellt und viermal mit Methylenchlorid extrahiert. Die organischen Phasen wurden über Natriumsulfat getrocknet, eingedampft und der Rückstand in 70 ml Methanol aufgenommen. Anschliessend wurde mit 0,8 g Palladium 5 % auf Kohle versetzt und bei Raumtemperatur und Normaldruck hydriert. Die Wasserstoff-Aufnahme war nach 24 Std beendet und betrug 95 % der Theorie. Der Katalysator wurde abfiltriert, die Lösung eingedampft und der Rückstand destilliert. Dabei isolierte man als Hauptfraktion 2,78 g (73 %) farblosen R-(-)-3-Aminobuttersäuremethylester ($Sdp._{10}$ : 60° C). $[\alpha]_D^{25}$ (c = 1; EtOH) = -32,0°.

IR (Film): 3380/3300 ($NH_2$); 1735 ($COOCH_3$); 1595 ($NH_2$)
NMR ($CDCl_3$): 1,13 (d; J = 6,5 Hz; $CH_3$-CH; 3H); 1,49 (s; $NH_2$; 2H); 2,0 - 2,7 (m; -$CH_2$-; 2H); 3,37 (sex. mit Feinaufspaltung; J ca. 6,5 Hz; -CH-; 1H); 3,67 (s; $OCH_3$; 3H);

b) Analog erhielt man aus dem zweimal umkristallisierten Hydrochlorid des SS-Methylesters den entsprechenden S-(+)-3-Aminobuttersäuremethylester in 73 % Ausbeute.
$[\alpha]^{25}_D$ (c = 1; EtOH) = +32,2°.

1.5 Herstellung von 3-Aminobuttersäuren

a) 700 mg (5,98 mMol) des R-(-)-Methylesters wurden in 8 ml konz. Salzsäure gegeben und für 5 Std bei 85° C gehalten. Das Reaktionsgemisch wurde eingedampft, in 2 ml Methanol gelöst und langsam mit 1,11 g (5,98 mMol) Tributylamin versetzt. Die Fällung wurde durch Zugabe von 15 ml Methylenchlorid vervollständigt. Abnutschen und Trocknen des Festkörpers ergab schliesslich 550 mg (89 %) farblose R-(-)-3- Aminobuttersäure. Smp. = 214 - 215° C.
$[\alpha]^{25}_D$ (c = 1; $H_2O$) = -34,8° (optische Reinheit 90 %)

b) Analog wurde aus dem S-(+)-Methylester die entsprechende S-(+)-3-Aminobuttersäure erhalten. Ausbeute: 80 %, Smp. = 215° C; $[\alpha]_D^{25}$ (c = 1; $H_2O$) = -36,5° (optische Reinheit 94 %).

**Beispiel 2**

**Herstellung des R-3-Aminopentansäuremethylesters**

2.1 Herstellung von 3-(1'-Methylbenzylamino)-2Z-pentensäuremethylester

62,4 g (0,47 Mol) 3-Oxopentansäuremethylester und 54,5 g (0,45 Mol) R-(+)-1-Phenylethylamin wurden mit 1,0 g Essigsäure in 150 ml Toluol 3 Stunden am Rückfluss erhitzt. Das Reaktionswasser (8,1 ml = 100 % der Theorie) wurde über einen Wasserabscheider entfernt.

Die Lösung wurde dann am Rotationsverdampfer eingeengt und schliesslich am Hochvakuum destilliert.

Als Hauptfraktion ($Sdp._{0,07}$ : 118° C) isolierte man 94,2 g Produkt mit einer Reinheit von 99,6 %.

UV (in MeOH): $\lambda_{max.}$ = 289 nm ($\varepsilon_{max.}$ = 10010)
IR (dünne Schicht) [$cm^{-1}$] : 3280 (NH), 1660 (CO, Ester) 1600 (Aromat, C = C)
NMR ($CDCl_3$) 300 Mhz: 1,0 (t; J = 7,5 Hz; $3CH_3$); 1,52 (d; J = 6,8 Hz; $3CH_3$); 1,99 (-$CH_2$-); 2,16 (-$CH_2$); 3,67 (s, Esterprot); 4,52 (s, Olefinprot); 4,67 (Methinprot); 7,35 - 7,2 (aromat.H); 9,03 (d, breit, 1NH-Prot).

2.2 Herstellung von 3-(1'-Methylbenzylamino)-pentansäuremethylester

25,0 g (0,102 Mol) 3-(1'-Methylbenzylamino)-2Z-pentensäuremethylester wurden in 100 ml abs. Methanol gelöst, mit 1,0 g Platin auf Kohle 5 % versetzt und bei Raumtemperatur und Normaldruck hydriert. Nach 24 Stunden war die Wasserstoffabsorption beendet (2,9 l). Man filtrierte vom Katalysator ab, dampfte die Reaktionslösung ein und destillierte den Rückstand im Hochvakuum.

Als Hauptfraktion ($Sdp._{0,3}$ : 75° C) wurden 21,3 g Produkt mit einer Reinheit von 99,2 % erhalten.

IR (dünne Schicht) [$cm^{-1}$]: 3340 (NH); 1730 (CO, Ester); 1600 (Aromat C = C)
NMR ($CDCl_3$) 300 Mhz: 0,85 (t; J = 7,4 Hz; $3CH_3$ Prot);
1,31 (d; J = 6,6 Hz; $3CH_3$ Prot);
1,40 (2-$CH_2$- Prot);
1,48 (s, breit; 1NH-Prot);
2,32 ($2CH_3$ vom RS Isomer);
2,43 (2-$CH_2$- vom RR Isomer);
2,72 (1 Methinprot);
3,61 (s; 3 Esterprot vom RS Isomer);
3,66 (s; 3 Esterprot vom RR Isomer);
3,87 (q; 1 Methinprot; J = 6,5 Hz);

7,4 - 7,18 (5 aromat.Prot).

2.3 Herstellung und Umkristallisation des Hydrochlorids des 3-(1'-Methylbenzylamino)-pentansäuremethylesters

28,6 g des rohen Diastereomergemisches Methylesters wurden in 410 ml Ether gelöst und die Lösung unter Eiskühlung mit gasförmiger Salzsäure gesättigt. Der Kristallbrei wurde abgenutscht und getrocknet. Man isolierte 31,0 g des Hydrochlorids des 3-(1'-Methylbenzylamino)- pentansäuremethylesters als Diastereomergemisch vom Smp. 174 - 174°C. 31,0 g des rohen Hydrochlorids wurden in 50 ml Methanol heiss gelöst und durch Zugabe von 160 ml Aceton zur Ausfällung gebracht.

Man erhielt 9,9 g weisses Produkt vom Smp. 195 - 196°C. 2.4 Herstellung des R-(-)-3-Aminopentansäureester

9,8 g (0,036 Mol) des umkristallisierten Hydrochlorids des RR-Methylesters wurden in 50 ml Wasser gelöst, mit Ammoniak alkalisch gestellt und viermal mit je 50 ml Methylenchlorid extrahiert. Die organischen Phasen wurden über Natriumsulfat getrocknet, eingedampft und der Rückstand in 120 ml Methanol aufgenommen. Anschliessend wurde mit 1,2 g Palladium auf Kohle 5 % versetzt und bei Raumtemperatur und Normaldruck hydriert. Die Wasserstoffaufnahme war nach 38 Stunden beendet. Der Katalysator wurde abfiltriert, die Lösung eingedampft und der Rückstand destilliert.

Dabei isolierte man als Hauptfraktion ($Sdp._4$ : 52 - 57°C) 3,6 g Produkt mit einem Gehalt von 88,9 %.

Die optische Drehung der Fraktion betrug $[\alpha]_D^{25}$ = -26,1° (c = 1, EtOH).

IR [$cm^{-1}$]: 3385, 3300 (NH); 1733 (CO, Ester); 1595 (NH); 840 (NH wagging)
NMR ($CDCl_3$) 300 Mhz: 0,94 (t; J = 7,2 Hz; $3CH_3$ Prot);
1,43 ($2-CH_2$-Prot);
1,45 (s, breit; 2NH-Prot);
2,26 ($1-CH_2$-Prot; J = 9 Hz);
2,48 ($1-CH_2$-Prot);
3,11 (1-Methin Prot);
3,69 (s; 3 Esterprot).

**Beispiel 3**

**Herstellung von R-(-)-3-Aminohexansäureethylester**

3.1 Herstellung des 3-(1'-Methylbenzylamino)-2Z-hexensäureethylesters

76,6 g (0,47 Mol) Butyrylessigsäureethylester, 54,5 g (0,45 Mol) R-(+)-1-phenylethylamin und 1,0 g konz. Essigsäure wurden in 150 ml Toluol gelöst und 3 Stunden am Rückfluss erhitzt.

Das Reaktionswasser (8,5 ml) wurde über einen Wasserabscheider entfernt. Anschliessend wurde die Lösung am Rotationsverdampfer eingeengt. Als Rückstand blieben 125,6 g farblose Lösung (Rohprodukt).

125,6 g dieses Rohproduktes wurden im Hochvakuum destilliert. Als Hauptfraktion ($Sdp._{0,08}$ : 147°C) erhielt man 108,3 g Produkt mit einer Reinheit von 99,3 %.

UV (in EtOH): $\lambda_{max.}$ = 288 nm ($\varepsilon_{max}$ = 21212)
205 nm ($\varepsilon_{max.}$ = 10808)
IR (dünne Schicht) [$cm^{-1}$]: 3280 (NH); 1650 (CO Ester); 1600 (Aromat, C = C); 760, 700 (C-H Aromat)
NMR ($CDCl_3$) 300 Mhz: 0,87 (t; $3CH_3$ Prot; J = 7,5 Hz);
1,27 (t; 3 Esterprot; J = 8,0 Hz);
1,33 - 1,55 ($3-CH_3$ Prot.; J = 7,0 Hz);
1,51 (d; $3CH_3$ Prot; J = 6,8 Hz);
1,86 - 2,16 ($2-CH_2$-Prot),;
4,13 (q; 2 Esterprot);
4,50 (s; 1-olef.Prot);
4,64 (q; 1 Methinprot);
7,18 - 7,35 (5 aromat.Prot);
9,03 (d, breit; 1 NH Prot).

3.2 Herstellung des 3-(1'-Methylbenzylamino) -hexansäureethylesters

52,2 g (0,1 Mol) 3-(1'-Methylbenzylamino)-2Z-hexensäureethylester wurden in 200 ml abs. Ethanol gelöst, mit 2,0 g Platin auf Kohle 5 % versetzt und bei Raumtemperatur und Normaldruck hydriert. Nach 27 Stunden war die Wasserstoffaufnahme beendet (4,25 l). Man filtrierte vom Katalysator ab, dampfte die Reaktionslösung ein und destillierte den Rückstand im Hochvakuum. Man erhielt:

1. Fraktion 0,6 g GC: 81,2 % $Sdp_{0,05}$ = 82°C
2. Fraktion 5,7 g GC: 86,5 % $Sdp_{0,05}$ = 95°C
3. Fraktion 37,7 g GC: 97,4 % $Sdp_{0,04}$ = 105°C

4. Fraktion 3,9 g GC: 57,7 % $Sdp_{0,08}$ = 116°C

Dies entsprach 44,4 g 100 %-igem Produkt ≙ 84,3 % Ausbeute, bezogen auf 100 %-iges Edukt.

UV (in EtOH) $\lambda_{max.}$ = 289 nm ($\varepsilon_{max.}$ = 1106)
IR (dünne Schicht) [$cm^{-1}$]: 3320 (NH); 1730 (CO Ester); 1600 (C = C, Aromat); 760, 700 (C-H Aromat);
NMR ($CDCl_3$) 300 Mhz: 0,82 (t; $3CH_3$ von RR Isomer);
0,88 (t; $3CH_3$ von RS Isomer);
1,27 (t; 3 Esterprot);
1,20 - 1,52 (4-$CH_2$-Prot);
1,32 (d; $3CH_3$ Prot);
1,50 (s, breit; 1NH Prot);
2,27 - 2,50 (2-$CH_2$-Prot);
2,77 (1 Methinprot);
3,88 (q; 1 Methinprot);
4,13 (q; 2 Esterprot);
7,18 - 7,36 (5 aromat. Prot).

3.3 Herstellung und Umkristallisation des 3-(1'-Methylbenzylamino)-hexansäureethylesters
36,4 g des rohen Diastereomergemisches des Ethylesters wurden in 460 ml Ether gelöst und die Lösung unter Eiskühlung mit gasförmiger Salzsäure gesättigt. Der Kristallbrei wurde abgenutscht und getrocknet.
Man isolierte 29,7 g des Hydrochlorids des 3-(1'-Methylbenzylamino)-hexansäureethylesters als Diastereomergemisch vom Smp. 126 - 130°C. 28,6 g des rohen Hydrochlorids wurden heiss aus 25 ml Ethanol umkristallisiert.
Man erhielt 15,0 g Produkt vom Smp. 138 - 139°C.
15,0 g des umkristallisierten Hydrochlorids wurden nochmals heiss aus 15 ml Ethanol umkristallisiert.
Man erhielt 12,6 g weisses Produkt vom Smp. 141 - 142°C. 3.4 Herstellung des R-(-)-3-Aminohexansäureethylesters
12,5 g des zweimal umkristallisierten Hydrochlorids des RR-Ethylesters wurden in 75 ml Wasser gelöst, mit Ammoniak alkalisch gestellt und viermal mit je 50 ml Methylenchlorid extrahiert. Die organischen Phasen wurden über Natriumsulfat getrocknet, eingedampft und der Rückstand in 140 ml Ethanol aufgenommen, Anschliessend wurde mit 1,4 g Palladium auf Kohle 5 % versetzt und bei Raumtemperatur und Normaldruck hydriert. Die Wasserstoffaufnahme war nach 38 Stunden beendet. Der Katalysator wurde abfiltriert, die Lösung eingedampft und der Rückstand destilliert. Dabei isolierte man:

1. Fraktion 1,01 g GC: 88,7 % $Sdp._2$ 55°C
2. Fraktion 3,62 g GC: 95,5 % $Sdp._2$ 57°C
3. Fraktion 0,30 g GC: 96,6 % $Sdp._2$ 56°C

Dies entsprach 4,65 g 100 %-igem Produkt ≙ 70,0 % Ausbeute. Die optische Drehung der 2. Fraktion betrug $[\alpha]_D^{25}$ = -23,2°. (c = 1, EtOH).

IR [$cm^{-1}$]: 3380, (NH-); 1734 (CO, Ester); 1595 (NH); 840 (NH wagging);
NMR ($CDCl_3$) 300 Mhz: 0,94 (3-$CH_3$ Prot);
1,27 (t; 3 Esterprot);
1,30 - 1,50 (4-$CH_2$-Prot);
1,43 (s, breit; 2NH Prot);
2,24 (1-$CH_2$-Prot);
2,46 (1-$CH_2$-Prot);
3,13 - 3,24 (1 Methinprot);
4,16 (q; 2 Esterprot; J = 7,1 Hz).

**Beispiel 4**

**Herstellung von R-(-)-3-Amino-4-benzylbuttersäuremethylester**

4.1 Herstellung des 3-(1'-Methylbenzvlamino)-4-benzyl-2Z-butensäuremethylesters
40,5 g (0,2 Mol) 3-Oxo-4-benzylbuttersäuremethylester, 24,2 g (0,2 Mol) R-(+)-1-Phenylethylamin und 1,0 g konz. Essigsäure wurden in 100 ml Toluol gelöst und 1,5 Stunden am Rückfluss erhitzt. Das Reaktionswasser (3,6 ml) wurde über einen Wasserabscheider entfernt. Anschliessend wurde die Lösung am Rotationsverdampfer eingeengt. Der Rückstand wurde über Kieselgel mit Ether/Petrolether 1 : 1 filtriert.
Man erhielt 66,85 g Rohprodukt (GC: 89,2 %). Dies entsprach 59,6 g 100 %-iges Produkt ≙ 96,3 % Ausbeute, bezogen auf 100 %-iges R-(+)-1-Phenylethylamin.

UV (in EtOH): $\lambda_{max.}$ = 289 nm ($\varepsilon_{max.}$ = 18914)
205 nm ($\varepsilon_{max.}$ 13339)
IR (dünne Schicht) [$cm^{-1}$]: 3280 (NH); 1655 (CO, Ester); 1605 (C = C, Aromat) 790, 700 (C-H, Aromat)
NMR ($CDCl_3$) 300 Mhz: 1,47 (d; 3-$CH_3$ Prot);
2,19 - 2,44 (2-$CH_2$-Prot);
2,52 - 2,78 (2-$CH_2$-Prot);
3,67 (s; 3 Esterprot);
4,55 (q; 1 Methinprot);
4,58 (s; 1 olef. Prot);
6,97 - 7,34 (10 aromat. Prot);
9,06 (d, breit; 1NH-Prot).

4.2 Herstellung des 3-(1'-Methylbenzylamino)-4-benzylbuttersäuremethylesters

15,3 g (0,049 Mol) 3-(1'-Methylbenzylamino)-4-benzyl-2Z-butensäuremethylester wurden in 50 ml abs. Methanol gelöst, mit 0,5 g Platin auf Kohle 5 % versetzt und bei Raumtemperatur und Normaldruck hydriert. Nach 60 Stunden war die Wasserstoffaufnahme beendet (1,2 l). Man filtrierte vom Katalysator ab, dampfte die Reaktionslösung ein und destillierte den Rückstand im Hochvakuum.

Man erhielt 13,8 g Produkt (GC: 93,2 %, $Sdp._{0,3}$ : 156° C). Dies entsprach 12,9 g 100 %-igem Produkt ≙ 83,8 % Ausbeute.

NMR ($CDCl_3$) 300 Mhz: 1,38 (d; $3HC_3$ Prot von RS Isomer);
1,41 (d; $3CH_3$ Prot von RR Isomer);
1,70 (s, breit; 1NH Prot);
1,70 - 1,95 (2-$CH_2$-Prot);
2,44 - 3,00 (4-$CH_2$-; 1 Methinprot);
3,62 (s; 3 Esterprot von RS Isomer);
3,65 (s; 3 Esterprot von RR Isomer);
3,87 (quintett, 1 Methinprot);
7,01 - 7,38 (10 aromat. Prot).

4.3 Herstellung und Umkristallisation des Hydrochlorids des 3-(1'-Methylbenzylamino)-4-benzylbuttersäuremethylesters

13,8 g des rohen Diastereomergemisches des Methylesters wurden in 150 ml Ether gelöst und die Lösung unter Eiskühlung mit gasförmiger Salzsäure gesättigt. Der Kristallbrei wurde abgenutscht und getrocknet. Man isolierte 12,3 g des Hydrochlorids als Diastereomergemisch vom Smp. 177 - 180° C. 12,2 g dieses Produktes wurden heiss in 27 ml Methanol gelöst und durch Zugabe von 75 ml Aceton zur Fällung gebracht.

Man erhielt 6,3 g Produkt mit einem Smp. von 189 - 190° C. 4.4 Herstellung des R-(-)-3-Amino-4-benzylbuttersäuremethylesters

6,2 g des umkristallisierten Hydrochlorids des RR-Methylesters wurden in 50 ml Wasser gelöst, mit Ammoniak alkalisch gestellt und viermal mit je 50 ml Methylenchlorid extrahiert. Die organischen Phasen wurden über Natriumsulfat getrocknet, eingedampft und der Rückstand in 60 ml abs. Methanol aufgenommen. Anschliessend wurde mit 0,6 g Palladium auf Kohle 5 % versetzt und bei Raumtemperatur und Normaldruck hydriert. Die Wasserstoffaufnahme war nach 60 Stunden beendet. Der Katalysator wurde abfiltriert, die Lösung eingedampft und der Rückstand destilliert. Man erhielt:

1. Fraktion 0,9 g GC: 93,5 % $Sdp._{0,2}$ : 98° C
2. Fraktion 1,4 g GC: 95,4 % $Sdp._{0,2}$ : 100° C
3. Fraktion 0,1 g GC: 93,7 % $Sdp._{0,1}$ : 95° C

Dies entsprach 2,3 g 100 %-igem Produkt ≙ 60,8 % Ausbeute. Die optische Drehung der 2. Fraktion betrug $[\alpha]_D^{25}$ = -2,3° (c = 1, EtOH).

UV (in EtOH): $\lambda_{max.}$ = 208 nm ($\varepsilon_{max.}$ = 8414)
IR (dünne Schicht) [$cm^{-1}$]: 3390 (NH); 1735 (CO Ester); 1600 (C = C Aromat); 1590 (NH); 750, 700 (C-H Aromat);
NMR ($CDCl_3$) 300 Mhz: 1,45 (s, breit; 2NH Prot);
1,58 - 1,82 (2-$CH_2$-Prot);
2,31 (1-$CH_2$-Prot);
2,50 (1-$CH_2$-Prot);
2,58 - 2,80 (2-$CH_2$-Prot);
3,21 (1 Methinprot);
3,68 (s; 3 Esterprot);
7,14 - 7,33 (5 aromat. Prot).

## Patentansprüche

1. Verfahren zur Herstellung optisch aktiver 3-Aminocarbonsäuren bzw. deren Ester aus β-Ketosäureestern, dadurch gekennzeichnet, daß man β-Ketosäureester mit chiralen Aminen in die entsprechenden Enamine überführt, diese durch Hydrierung in Gegenwart eines platinkatalysators in die entsprechenden N-substituierten Aminosäureester überführt, letztere durch Einleiten von HCl-Gas in die Hydrochloride überführt, durch Umkristallisation und Umfällen die gewünschten diastereomeren N-substituierten Aminosäureesterhydrochloride abtrennt, diese neutralisiert und aus diesen neutralisierten Produkten durch Hydrogenolyse in Gegenwart eines palladiumkatalysators die gewünschten optisch aktiven 3-Aminocarbonsäureester freisetzt und isoliert oder durch Verseifung in die Säuren überführt.

2. Verfahren nach Anspruch 1, dadurch gekennzeichnet, daß man als chirales Amin Phenylethylamin verwendet.

3. Verfahren nach einem der Ansprüche 1 oder 2, dadurch gekennzeichnet, daß man einen Platinkatalysator, der 2 bis 10 % Platin auf einem Trägermaterial aufweist, in einer Menge von 3 bis 5 Gew.-%, bezogen auf eingesetztes Enamin, verwendet.

4. Verfahren nach einem der Ansprüche 1 bis 3, dadurch gekennzeichnet, daß man einen Palladiumkatalysator, der 2 bis 10 % Palladium auf einem Trägermaterial aufweist, in einer Menge von 9 bis 15 Gew.-%, bezogen auf eingesetzten N-substituierten Aminocarbonsäureester, verwendet.

## Claims

1. Process for preparing optically active 3-aminocarboxylic acids and their esters resp. from β-keto acid esters, characterized in that β-keto acid esters are transformed into the corresponding enamines with chiral amines, said enamines are transformed into the corresponding N-substituted amino acid esters by hydrogenation in the presence of a platinum catalyst, the amino acid esters are transformed into the hydrochlorides by introduction of HCl gas, the desired diastereomeric N-substituted amino acid ester hydrochlorides are separated by recrystallisation and reprecipitation, said amino acid ester hydrochlorides are neutralized and the desired optically active 3-aminocarboxylic acid esters are liberated from these neutralized products by hydrogenolysis in the presence of a palladium catalyst and isolated or converted into the acids by saponification.

2. Process according to claim 1, characterized in that as chiral amine phenyl ethyl amine is used.

3. Process according to any one of claims 1 or 2, characterized in that a platinum catalyst showing 2 to 10 % of platinum on a support material is used in an amount of 3 to 5 weight-%, based on the enamine used.

4. Process according to any one of claims 1 to 3, characterized in that a palladium catalyst showing 2 to 10 % of palladium on a support material is used in an amount of 9 to 15 weight-%, based on the N-substituted aminocarboxylic acid ester used.

## Revendications

1. Procédé pour la préparation d'acides 3-amino-carboxyliques optiquement actifs ou respectivement de leurs esters à partir d'esters d'acides β-cétoniques, caractérisé en ce qu'on transforme des esters d'acides β-cétoniques au moyen d'amines chirales en les énamines correspondantes, on transforme celles-ci par hydrogénation en présence d'un catalyseur à base de platine en les esters d'aminoacides N-substitués correspondants, on transforme ces derniers en les chlorhydrates par introduction de HCl gazeux, par recristallisation et précipitation, on sépare les chlorhydrates d'esters d'aminoacides N-substitués diastéréoisoméres recherchés, on neutralise ceux-ci et à partir de ces produits neutralisés, on libère les esters d'acides 3-aminocarboxyliques optiquement actifs recherchés par hydrogénolyse en présence d'un catalyseur à base de palladium et on les isole ou les transforme en les acides par saponification.

2. Procédé selon la revendication 1, caractérisé en ce que l'on utilise, comme amine chirale, la phényléthylamine.

3. Procédé selon l'une des revendications 1 ou 2, caractérisé en ce qu'on utilise un catalyseur à base de platine qui présente 2 à 10 % de platine sur un matériau support, en une quantité de 3 à 5 % en poids, par rapport à l'énamine mise en oeuvre.

4. Procédé selon l'une des revendications 1 à 3, caractérisé en ce qu'on utilise un catalyseur à base de palladium qui présente 2 à 10 % de palladium sur un matériau support, en une quantité de 9 à 15 % en poids par rapport à l'ester d'acide aminocarboxylique N-substitué mis en oeuvre.